# EUROPEAN PATENT APPLICATION

(11) **EP 1 217 371 A1**
(43) Date of publication of application: **26.06.2002**
(21) Application number: 00610139.8
(22) Date of filing: 22.12.2000
(51) Int. Cl.: G01N 30/88

(54) **An improved method for quantitative assessment of anthropogenic compounds in soil**

(71) Applicant: A/S AnalyCen, 7000 Fredericia (DK)
(72) Inventor: Ivarsson, Per Reinhold Josef, 531 97 Lidköping (SE)
(74) Representative: Christensen, Bent

(57) **Abstract**

In a method for quantitative assessment of anthropogenic compounds in soil a more accurate estimate of the degree of contamination in a soil sample is obtained by performing a chromatographic separation followed by mass spectrometry (MS) analysis of an extract of the sample, wherein the analysis results are processed in such a way that contributions from naturally occurring terpenes are excluded from the total amount of aliphatic hydrocarbon compounds in the extract. Further, the method of the invention makes it possible to assess the total amount of alkyl substituted benzene compounds present in the soil sample as well as the amount of alkyl substituted benzene compounds not comprising terpene moieties present in the soil sample. Finally the method of the invention makes it possible to assess the amount of terpenes present in the sample.

## Description

### Technical field

The present invention relates to a method for quantitative assessment of anthropogenic compounds in soil.

### Background art

Governments around the world are becoming increasingly aware of the problems associated with environmentally hazardous materials detectable in the environment surrounding us, and in most countries governmental authorities have provided legislation regulating the allowable threshold limits for these materials.

Concerning soil contamination, in Denmark for example, regional authorities are making surveys concerning the historical background for selected sites. That is, in order to be able to predict potentially contaminated sites it is investigated whether or not potentially environmentally hazardous activities or companies (such as petrol stations or automobile repair shops, refineries, garages, oil depots etc.) have ever taken place or been located on said sites. When a potentially contaminated site is spotted, an analysis is carried out based on soil samples from said site, and if it turns out that the contamination of these soil samples exceeds the allowed threshold limit, this fact is to be entered into the land registry registers. If it further turns out, that the site is polluted to the extend that the subsoil water may be contaminated, the authorities must order the proprietor of the site in question to perform a purification treatment of the soil. However, it is common practice simply to remove the upper layers of the contaminated soil and to deposit the soil at authorized deposition lots.

Nevertheless, both of these solutions are very expensive. Thus, when such radical measures are conducted it is of course extremely important that the analysis result really produces an accurate picture concerning the amount of the contamination and that no false contribution to the amount of contamination in the analysis result is present (such as plant material contributing to the overall hydrocarbon content), so that the purification treatment of the soil or the removal and deposition of the soil at remote sites is only conducted when the soil indeed is contaminated. As an example some regional and local authorities in Denmark are classifying and restricting allowable application of contaminated soil in accordance with analysis results obtainable by the method of prior art. These classifications are as follows: Class 1: uncontaminated, all possible applications - no limitation; Class 2: slightly contaminated, the soil may be used for construction work; Class 3: contaminated, the soil must be deposited at authorized deposition lots or must be purified; Class 4: heavily contaminated, the soil must be deposited at special authorized deposition lots under more strict conditions or must be purified.

Currently a number of different methods for the determination of anthropogenic compounds in soils, such as oil contaminations, are applied. These methods may be divided in two major groups, viz. analysis methods performed in the field and laboratory analysis methods.

The first group comprises primarily immuno chemical methods, fluorescence/UV methods and photoionising detecting methods. Although these methods are very convenient for doing measurements in the field, the results of these methods are generally considered relatively doubtful. Thus, more accurate analyses are often required and these are to a wide extend carried out in an accredited laboratory.

The laboratory analyses commonly performed are 1) freon extraction followed by IR-spectroscopy and 2) extraction with organic solvent followed by analysis employing a gas chromatography-flame ionisation detector (GC-FID). The first of these laboratory analysis methods does not give any information as to either the composition or the type of the anthropogenic substances, such as oils. However, this method gives information concerning the amount of hydrocarbons, including naturally occurring hydrocarbons. By using the second type of laboratory analysis methods mentioned above, ie. the GC-FID method - and by comparison with standard solutions of known substances - it is possible to gain information concerning the type of compounds present (ie. amount of aliphatic and aromatic compounds), but it is not possible in this method to distinguish naturally occurring terpenes, which are often found in plant material, from anthropogenic compounds originating from eg. oils.

The classification above is made on the basis of GC-FID analyses. Thus, if naturally occurring hydrocarbon compounds are present in the soil sample, the soil might get a higher classification number resulting in the need to deposit or purify soil that should not have been ranked that high.

For references to the methods of prior art see: VKI's report (VKI is the reference laboratory of the Danish Environmental Protection Agency): "Bestemmelse af olie i jord, Gaschromatografisk metode" (English: *"The Determination of Oil in Soil, Gaschromatographic Method*", July 1998; ISO TR 11046 "Soil Quality - Determination af Mineral Oil Content - Method by Infrared Spectrometry and Gas Chromatographic Method", 01.06.1994; Technical Report, Interlaboratory Study of Three Methods for Analyzing Petroleum Hydrocarbons in Soil. American Petroleum Institute, 4599, 1994; "Nordic Interlaboratory Test", Report of the Danish Environmental Protection Agency No. 30, ISBN 87-7810-777-6, 1997.

In Sweden the authorities require that the analysis of soil samples are quantified according to type of compound. That is, the amounts in the soil of > C₅-C₈-aliphatics; > C₈-C₁₀-aliphatics; > C₁₀-C₁₂-aliphatics; > C₁₂-C₁₆-aliphatics; > C₁₆-C₃₅-aliphatics; benzene; toluene plus ethylbenzene plus xylenes; > C₈-C₁₀-aromatics and > C₁₀-C₃₅-aromatics respectively are to be reported separately, and allowable maximum values exist for each of these compounds types.

Accordingly, by the analysis methods of prior art it is not possible in a soil sample to distinguish naturally occurring hydrocarbons from hydrocarbons originating from anthropogenic material, such as oil. Examples of anthropogenic compounds which may be difficult to distinguish from naturally occurring hydrocarbons are eg. aliphatic and aromatic oils having 10-40 carbon atoms. These include for example light gas oil, fuel oil, paraffin oil, heavy gas oil, motor oil etc. The type of oil may be determined from its chromatographic pattern, but mixtures and partially decomposed oils which are often present in contaminated soils may be excluded from being identified in this way.

Terpene compounds is a class of chemical compounds having moieties comprising the isoprene structure and it is well known that this class of compounds is widely abundant in plant material. Plant material may contain 1-20 wt% of terpenes and may accordingly contribute substantial amounts of hydrocarbons to the soil.

Thus, by the analysis methods of prior art it is in certain instances inevitably that contributions originating from non-hazardous plant material is comprised in the analysis result of the amount of hydrocarbon compounds. This means that soil in these instances unnecessarily have to be removed to deposition sites or even worked up by purification methods due to the fact that eg. non-hazardous terpene compounds originating from plant material add a "false" contribution to the overall hydrocarbon content when the amount of contamination of the soil is to be determined.

Thus, prior to the present invention a method for the quantitative determination of the hydrocarbon content in soil excluding "false" contributions from terpenes originating from plant material, was needed.

It has now been found that it is possible in a very simple and inexpensive manner to determine the amount of aliphatic compounds in a soil sample by using a chromatographic separation method combined with a mass spectrometer wherein the obtained data are processed in such a way, that the contribution from terpenes is subtracted from the overall content of hydrocarbon compounds; giving a more accurate quantitative assessment of anthropogenic compounds present in the soil. Thus, by the method according to the present invention it is possible to assess the amount of anthropogenic aliphatic as well as aromatic hydrocarbon compounds in a soil sample. It is also possible to assess the amount of terpene compounds - which very likely originate from plant material - present in the soil sample.

### Brief description of the invention

Accordingly the present invention relates to a method for quantitative assessment of anthropogenic compounds in a soil sample, wherein
1) an extract of the soil sample is subjected to a method for chromatographic separation of individual components in the extract; and
2) MS analyses are conducted on individual components found in step 1); and
3) the chromatograms corresponding to mass spectra in the MS analyses which show presence of
   a) one or more of the ions m/z = 43, *m*/*z* = 57, *m*/*z* = 71, *m*/*z* = 85
   b) one or more of the ions *m*/*z* = 77, *m*/*z* = 91, *m*/*z* = 105
   c) one or more of the ions *m*/*z* = 68, *m*/*z* = 69, *m*/*z* = 93, *m*/*z* = 119
   respectively are identified and peak integration of each of these three chromatograms are performed; and
4) peak areas and retention times for individual peaks in each of the chromatograms
   a) identified in step 3a)
   b) identified in step 3b)
   c) identified in step 3c)
   respectively are listet numerically; and
5a) one or more peaks in the chromatogram identified in step 3a) which with respect to retention time coincide with one or more of the peaks corresponding to one or more of the ions set forth in step 3b) and/or step 3c) are deleted from the list obtained in step 4a); and
5b) the concentration of aliphatic compounds in the soil sample extract is calculated as the sum of peak areas obtained from the revised list obtained in step 5a) relative to the sum of peak areas of a standard having a known concentration, and the concentration of aliphatic compounds in the soil sample is calculated on the basis of this; and/or
6) the concentration of alkyl substituted benzene compounds in the soil sample extract is calculated as the sum of peak areas obtained from the list obtained in step 4b) relative to the sum of peak areas of a standard having a known concentration, and the concentration of alkyl substituted benzene compounds in the soil sample is calculated on the basis of this; and/or
7a) one or more peaks in the chromatogram identified in step 3b) which with respect to retention time coincide with one or more of the peaks corresponding to one or more of the ions set forth in step 3c) are deleted from the list obtained in step 4b);
7b) the concentration of alkyl substituted benzene compounds not comprising terpene moieties present in the soil sample extract is calculated as the sum of peak areas obtained from the revised list obtained in step 7a) relative to the sum of peak areas of a standard having a known concentration, and the concentration of alkyl substituted benzene compounds not comprising terpene moieties present in the soil sample is calculated on the basis of this; and/or
8) the concentration of terpene compounds in the soil sample extract is calculated as the sum of peak areas obtained from the list obtained in step 4c) relative to the sum of peak areas of a standard, and the concentration of terpene compounds in the soil sample is calculated on the basis of this.

The present invention is based on the finding, that it is possible to obtain a more accurate assessment of the amount of contamination in a soil sample by performing a chromatographic separation method followed by mass spectrometry (MS) analysis on an extract of the soil sample, wherein the obtained analysis results are processed in such a way, that contributions from naturally occurring terpenes are excluded from the total amount of aliphatic hydrocarbon compounds in the extract. Thus, a more accurate picture of the amount of anthropogenic hydrocarbon compounds in the soil sample is obtained by excluding the hydrocarbon contribution originating from terpenes. Moreover, by the method of the present invention it is possible to assess the total amount of alkyl substituted benzene compounds present in the soil sample as well as the amount of alkyl substituted benzene compounds not comprising terpene moieties present in the soil sample. And finally it is possible to assess the amount of terpenes present in the soil sample.

### Drawings

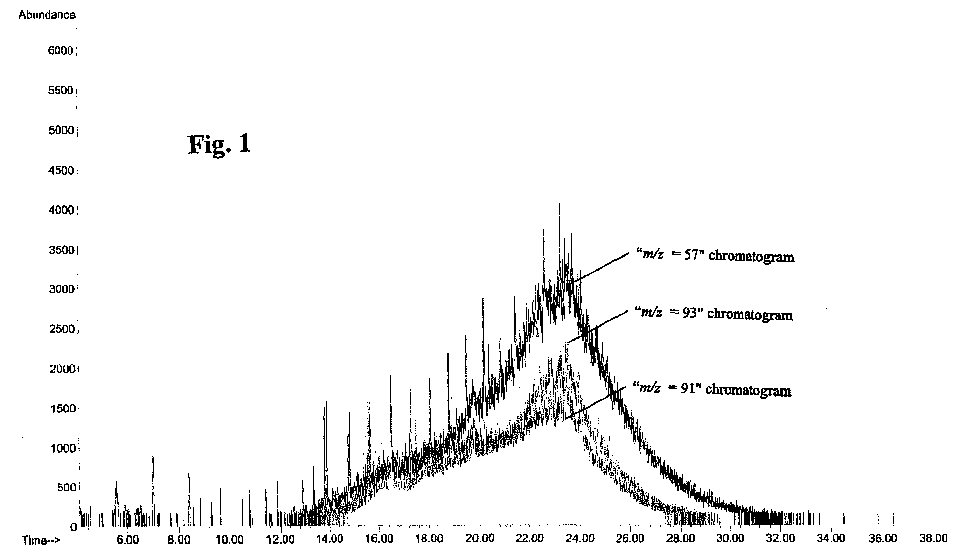

Fig.1 shows the "ion-chromatograms"of the ions *m*/*z* = 57, *m*/*z* = 91, *m*/*z* = 93 respectively from an analysis of an authentic soil sample according to the invention.

This soil sample was analysed in example 1 by the method according to the invention as well as by a prior art method.

### Detailed description of the invention

In the method of the present invention a soil sample extract is subjected to a chromatographic separation method followed by mass spectrometry. Examples of chromatographic separation methods that might be used in the method of the present invention are gas chromatography (GC) and high-pressure liquid chromatography (HPLC). At present the GC separation method is preferred due to the easy generation of EI-spectra, which is the preferred mode of ionisation. In the following section a description of the GC-MS procedure is given. However, a person skilled in the art will know how to adapt this description of the GC-MS procedure to a LC-MS procedure.

In a GC-MS analysis procedure a small amount of a solution of a sample is injected into a GC-MS apparatus. The injected sample is transported through a helical column by means of a carrier gas which typically is ultra pure helium. The column is heated in accordance with a predefined temperature program; the effect of this heating is that the compounds in the sample evaporate. The non-polar nature of the inside surface of the column causes the different compounds in the sample to move with different speeds so that at the end of the column the different compounds in the sample (or at least some of them) will be separated. At the end of the column a detector detects the intensity of matter leaving the column. The time span from entering the column to hitting the detector is called the retention time and this time value is characteristic for each compound (as well as for the type of column, the temperature of the column etc.). When the compounds leave the column they enter into the mass spectrometry part of the system, wherein the compounds are ionised and accelerated in a combined electrical or magnetic field. Due to their electrical charge the species will be deflected in a circular path and hit a detector which position correlates with the mass and the charge of the species. Thus, by detecting the radius of the circular path of a fragment the mass of that fragment may be determined. The masses may also be determined as the flight time of the fragments as in a time-of-flight (TOF) instrument or as the deflection in a cyclotron as in ion trap instruments.

However, in a MS instrument utilizing the electron impact (EI) technique for ionisation, the compound is ionised to its corresponding positive ion (also termed parent ion), but very many compounds also fragment in this process. This means that when a specific component corresponding to a specific compound exits the column and enters into the ionising part of the MS, a proportion of this component is simply ionised and continues through the combined electrical and magnetic fields where it finally will be detected as having a mass corresponding to this specific parent compound. However, another proportion of the component corresponding to the specific compound will fragment in the ionising process, so that different species having different masses appear. These fragmented species also continue through the combined electrical and magnetic fields and will be detected as having a mass corresponding to the mass of each specie respectively.

Accordingly, by using a GC-MS apparatus it is possible to separate a mixture into its components and to analyse each component of the mixture as to amount and retention time (in a so called GC-chromatogram) and gain information concerning the molecular weight of each compound in the mixture (in a so called mass spectrogram). Furthermore, by studying the fragmentation pattern (the abundances and masses of species appearing from fragmentation) of a compound it may in rare instances be possible to elucidate the structure of a compound in the sample.

GC-MS instruments are today automatized and computerized so that the sample is simply injected into the GC inlet and the apparatus is doing all the necessary work in accordance with the settings of the instrument. When the predefined time span for data sampling has lapsed, it is possible on a computer screen to retrieve chromatograms representing selected fragments detected in the mass spectrometer. It is also possible to subtract or add chromatograms representing different fragments detected in the mass spectrometer.

The present inventor has found that it is possible in a simple and inexpensive manner to obtain a more accurate assessment of the amount of anthropogenic compounds in a soil sample by excluding the contribution from terpenes to the overall hydrocarbon content.

One of the features of the present invention is the finding that terpenes display a distinct fragmentation pattern in mass spectra. That is, in mass spectrometry analysis of a terpene compound the ion *m*/*z* = 93 very often appears in the spectrum. This ion very likely corresponds to the fragment C₇H₉. Other ions often found for terpene compounds in MS spectra are the ions *m*/*z* = 68, *m*/*z* = 69 and *m*/*z* = 119. These ions very likely correspond to the fragments C₅H₈, C₃H₅CO, C₆H₅C₃H₆ respectively. These are also present in the spectrogram in relatively high abundances.

In a mass spectrogram aliphatic compounds display a fragmentation pattern in which the ions *m*/*z* = 43, *m*/*z* = 57, *m*/*z* = 71 and *m*/*z* = 85 respectively are present in relatively high abundances. These ions correspond to the fragments C₃H₇, C₄H₉, C₅H₁₁ and C₆H₁₃ respectively and simply appear by tearing apart the hydrocarbon chain into fragments of various lengths.

In a mass spectrogram alkyl substituted benzenes display a fragmentation pattern in which the ion *m*/*z* = 91 very often is present in a relatively high abundance. This ion corresponds to the fragment C₆H₅CH₂. Other ions very often present in the mass spectrogram of an alkyl benzene is the ions *m*/*z* = 77 and *m*/*z* = 105. These ions correspond to the fragments C₆H₅ and C₆H₅C₂H₄ respectively. It should be noted that the alkyl moiety of an alkyl substituted benzene may of course also fragment and give rise to a fragmentation pattern typically for aliphatic alkyl compounds as set forth above.

Accordingly, when carring out the method of the present invention the following steps are followed:

### 1. Chromatography

The soil sample is extracted with a suitable solvent, such as pentane, dichloromethane, toluene, hexane, ethyl acetate, acetone or mixtures thereof. The extraction process may involve high pressure and temperature, ultrasonication, microwaves, shaking etc. After the extraction process the suspension is preferable centrifuged and the organic phase is recovered for analysis. Preferably the organic phase is dried with a typically drying agent, such as sodium sulphate. The internal standard may be added to the solvent either prior to or after the extraction process.

An extract of the soil sample is then subjected to a method for chromatographic separation of individual components in the extract. Preferably gas chromatography is used, but other chromatographic means, such as liquid chromatography (LC), may be used as well.

### 2. Mass spectrometry

Mass spectrometry is conducted on individual components found in step 1. Mass spectrometry should be conducted in electron impact (EI) mode and ions monitored in scan or SIM mode.

### 3. Identification of the chromatograms corresponding to aliphatic, alkyl substituted benzene and terpene species in the mass spectra

The chromatograms corresponding to mass spectra in the MS analyses which show presence of
a) one or more of the ions *m*/*z* = 43, *m*/*z* = 57, *m*/*z* = 71, *m*/*z* = 85, all of which may relate to aliphatic species present in the sample;
b) one or more of the ions *m*/*z* = 77, *m*/*z* = 91, *m*/*z* = 105, all of which may relate to alkyl substituted benzene species present in the sample;
c) one or more of the ions *m*/*z* = 68, *m*/*z* = 69, *m*/*z* = 93, *m*/*z* = 119, all of which may relate to terpene species present in the sample
respectively are then identified and peak integration of each of these chromatograms are performed.

Preferable, only the chromatograms corresponding to mass spectra in the MS analyses which show presence of
a) one or two of the ions *m*/*z* = 57, *m*/*z* = 71
b) one or two of the ions *m*/*z* = 91, *m*/*z* = 105
c) one or two of the ions = 93, *m*/*z* = 119
respectively are identified whereafter peak integration is performed, and most preferred only the chromatograms corresponding to mass spectra in the MS analyses which show presence of
a) one or two of the ions *m*/*z* = 57
b) one or two of the ions *m*/*z* = 91
c) one or two of the ions = 93
respectively are identified whereafter peak integration is performed.

Preferable the signal should be at least 10 times the noise level when selecting the peaks that are to be included in the integration. Chromatograms for standard solutions having known compositions and concentrations are recorded as well for calibration purposes. The sample chromatograms and the standard chromatograms should be recorded with the same integration parameters.

The standards recorded for each type of compound (eg. aliphatic, alkyl substituted benzene and terpene compounds) of interest in the analysis method according to the present invention should be choosen so as to resemble the compounds present in the soil sample with respect to response factors. The smaller the difference between these response factors is (ie. the difference between response factor of the sample compounds and the standard compound(s)), the more accurate the analysis result will be. The standards used should contain the internal standard.

For assessing the amount of aliphatic compounds in the soil sample the chromatogram may be integrated against a product standard containing eg. motor oil, diesel oil etc. with a known concentration, or the integration could be made against a standard comprising paraffin compounds comprising C₁₀-C₃₆ aliphatics with a known concentration. When using eg. paraffin it is possible to assess the amount of aliphatic compounds in the soil sample that have specific chain lengths, eg. > C₁₀-C₁₂, > C₁₂-C₁₆, > C₁₆-C₃₆. However, also other compounds may be used as standards for quantification of the aliphatic compounds.

Examples of standard compounds that can be used for quantification of the terpenes present in the soil sample are farnesol, squalene and limonene and mixtures of these. However, any other suitable terpene or mixture of terpenes with a known concentration may be used. Also other types of compounds may be used, but as stated above, the accuracy of the assessment may decrease if the response factor of the standard differs too much relative to the response factor of the sample.

Examples of standard compounds that can be used for quantification of the alkyl substituted benzene compounds present in the soil sample are dipropyl benzenes, tripentylbenzenes, tetrahexylbenzenes and mixtures of these. However, any other suitable alkyl substituted benzene or mixture of alkyl substituted benzenes may be used. Also other types of compounds may be used as standard, but again - as stated above - the accuracy of the assessment may decrease if the response factor of the standard differs too much relative to the response factor of the sample.

### 4. Numerically listing of peak areas and retention times for individual peaks in the chromatograms identified in step 3

Peak areas and retention times for individual peaks in each of the chromatograms
a) identified in step 3a)
b) identified in step 3b)
c) identified in step 3c)
respectively are then listed numerically.

According to the method of the present invention it is possible to assess the amount of one or more of the following types of substances in the soil sample: aliphatic hydrocarbon compounds (as explained in steps 5a) and 5b)); total alkyl substituted benzenes (as explained in step 6)); alkyl substituted benzene compounds not comprising terpene moieties (as explained in step 7a) and 7b)); and finally terpene compounds (as explained in step 8)).

### 5. Calculation of the concentration of aliphatic hydrocarbon compounds in the soil sample

a) To exclude contribution from terpenes and alkyl substituted benzenes in the list obtained in step 4a), peaks in the chromatogram identified in step 3a) which with respect to retention time coincide with one or more of the peaks corresponding to one or more of the ions set forth in 3b) and/or step 3c) are deleted from the list obtained in step 4a). Thus a new list is obtained which essentially only represents peaks of aliphatic hydrocarbon compounds.
b) The concentration of aliphatic compounds in the soil sample extract is then calculated as the sum of peak areas obtained from the revised list obtained in step 5a) relative to the sum of peak areas of the standard sample having a known concentration, and the concentration of aliphatic hydrocarbon compounds in the soil sample can easily be calculated from this. Often different aliphatic hydrocarbons with different chain lenghts are quantified independently. A person skilled in the art will know how to do this.

In most cases the integration of of the peak areas are made relative to the zero-base line. However, sometimes a situation may arise wherein within specific range/ranges of retention times the chromatogram corresponding to aliphatic hydrocarbons, ie. the chromatogram identified in step 3a) and at least one of the curves corresponding to alkyl substituted benzenes, ie. the chromatogram identified in step 3b), or to terpene compounds, ie. the chromatogram identified in step 3c) for this/these specific range/ranges of retention times - when plottet together - display curves which within said specific range/ranges of retention times have essentially the shape of hills with peaks on top and wherein the hills have essentially the same curve form or shape but not necessarily the same intensity and wherein the different hills have their respective maxima at essentially the same retention time. This situation is due to the fact, that unresolved mass spectra are obtained, and this may occur if the soil sample has a high content of compounds that possess aliphatic carbon chains in combination with either terpene or alkylbenzene moieties or both. Accordingly in such a situation, the area under the curve of the chromatogram corresponding to the ion(s) set forth in step 3a) for this/these ranges of retention times does not correlate very accurately with the amount of aliphatic species present in the soil sample. Thus, in such a case, rather than performing the integration of the peaks of the chromatogram identified in step 3a) relative to the zero-base line this integration is performed on a valley-to-valley basis within this/these range/ranges of retention times. Fig. 1 shows an example of three chromatograms each representing a "hill" with peaks on top and thus a situation where the integration of the chromatogram identified in step 3a) should be performed on a valley-to-valley basis - at least for a certain range of retention times.

### 6. Calculation of the total concentration of alkyl substituted benzene compounds in the soil sample

The concentration of alkyl substituted benzene compounds in the soil sample extract is then calculated as the sum of peak areas obtained from the list obtained in step 4b) releative to the sum of peak areas of a standard having a known concentration, and the concentration of alkyl substituted benzene compounds in the soil sample can easily be calculated from this.

Alternative or in addition to the method of calculation of the total concentration of alkyl substituted benzene compounds in the soil sample, as described above in step 6), it is also possible to calculate the concentration of alkyl substituted benzene compounds not comprising terpene moieties present in the soil sample. This method is described below in steps 7a) and 7b).

### 7. Calculation of the concentration of alkyl substituted benzene compounds not comprising terpene moieties present in the soil sample

a) To exclude contribution from terpenes in the list obtained in step 4b), peaks in the chromatogram identified in step 3b) which with respect to retention time coincide with one or more of the peaks corresponding to one or more of the ions set forth in step 3c) are deleted from the list obtained in step 4b). Thus a new list is obtained which essentially only represents peaks of alkyl substituted benzene compounds not comprising terpene moieties;
b) The concentration of alkyl substituted benzene compounds not comprising terpene moieties present in the soil sample extract is then calculated as the sum of peak areas obtained from the revised list obtained in step 7a) relative to the sum of peak areas of a standard having a known concentration, and the concentration of alkyl substituted benzene compounds not comprising terpene moieties present in the soil sample is calculated on the basis of this; and/or

### 8. Calculation of the concentration of terpene compounds in the soil sample

The concentration of terpene compounds in the soil sample extract is calculated as the sum of peak areas obtained from the list obtained in step 4c) relative to the sum of peak areas of a standard, and the concentration of terpene compounds in the soil sample can easily be calculated from this.

Preferably, when selecting the peaks in the chromatogram identified in step 3a) to be deleted from the list obtained in step 4a), only those peaks in the chromatogram obtained in step 3a) that have coincidal peaks in the chromatogram obtained in step 3b) and/or 3c) having abundances of a certain size (eg. of at least 10%) of the value of the corresponding peak in the chromatogram identified in step 3a) respectively should be considered. Analogue considerations should be made when selecting the peaks to be deleted from the list obtained in step 4b) for the calculation of the amount of alkyl substituted benzene compounds not comprising terpene moieties in step 7).

### Examples

Two soil samples were subjected to the process of the present invention and the results were compared with the results of the GC-FID analysis technique according to prior art.

In the following examples the sample preparation for the GC-MS were made as follows: 20 g authentic soil sample was shaken in 1 hour with a buffer solution of 0.05 M sodium pyrophosphate and 10 ml hexane/ethyl acetate (1:1) with internal standard of o-terphenyl. The suspension was then centrifuged and the supernatant was dried with sodium sulphate.

The GS-MS analyses were conducted on an Agilent 6890/5973 instrument having a PE-5 column of PE-5 with a length of 20 m. The internal diameter of the column was 0.18 mm and the film thickness was 8 µm. The amount of injected sample was 1 µm and the carrier gas was helium having af flow rate of 0.7 ml/min. The temperature program was as follows: 60 °C in 3 minutes, increase of 7 °C/min. to 310 °C where it was held for 20 min. The mode of ionization was electron impact (EI) at 70 eV. The mass range recorded was 30-350.

The GC-FID method used in the following examples was the accredited method (OM Standard - Method KG.22A). The method was conducted as follows: The soil sample was extracted with pentane after addition of a pyrophosphate buffer. The extaction was performed by shaking for 2 h. The extract was then analysed by means of a GC with a flame ionisation detector (GC-FID). The quantification of hydrocarbons was performed against a known reference standard (BTEX standard mixture). The GC part was conducted with a HP 5 column of 25 m, having an internal diameter of 0.2 mm. The film thickness was 0,33 µm. 2 µl sample solution was injected. The carrier gas was hydrogen. The temperature program was as follows: 35 °C for 3 min, rising to 300 °C at 10 °C/min where it was held for 10 min.

### Example 1a

An authentic soil sample was subjected to the extraction treatment mentioned above and 1 µl was injected in the GC-MS instrument having the above settings.

When the sampling was accomplished a complete TIC (total ion chromatogram) was obtained. The TIC had the shape of a hill with peaks on top. Comparing the individual mass spectra found in the chromatogram with a selection of spectra of compounds in a computer database revealed that many terpenes and alkyl substituted benzene compounds were present.The "ion chromatogram" of the ions *m*/*z* = 93 and *m*/*z* = 91 respectively showed very high abundances of these ions compared to the chromatogram of the "57-ion" and thus high abundances of terpene and alkyl substituted benzene compounds. Fig. 1 display these three chromatograms which all appear to comprise "hills" with peaks on top. Due to this, the integration of peak areas in the "57-ion" chromatogram for the range of retention times within this "hill" was performed on a valley-to-valley basis. Retention times for the peaks in the "ion chromatograms" corresponding to the ions *m*/*z* = 57, *m*/*z* = 93 and *m*/*z* = 91 respectively were then listed numerically and the peaks in the list corresponding to the "57-ion" which with respect to retention time coincided with peaks in the the chromatograms corresponding to the "91-ion" and "93-ion" respectively were deleted from the "57-ion"-list, and the revised list thus obtained corresponded to the chromatogram of the compounds in the sample that are aliphatic hydrocarbon substances neither substituted with aromatic moieties nor terpene moieties. The amounts of aliphatic hydrocarbon substances with different chain lengths in the soil sample extract were then calculated as the sum of peak areas relative to the sum of peak areas of the standard of paraffin standard in a usual manner, and finally the concentrations of aliphatic hydrocarbons in the soil sample were calculated. The amount of alkyl substituted benzenes as well as the amount of terpene compounds was calculated by quantifying the sum of peak areas of the "91-ion" and "93-ion" chromatograms respectively against a standard of ortho-terphenyl. The results from example 1a is listed in table 1 below.

### Example 1b - Comparison

An identical sample preparation as in example 1a was used for analysis according to the GC-FID method in accordance with the conditions set forth above. The results from example 1b is listed in table 1 below.

**Table 1**

| **Chain length** | **Example 1a Method according to the invention (mg/kg)** | **Example 1b GC-FID method (prior art) (mg/kg)** |
|---|---|---|
| Amount of aliphatic compounds in the soil sample of example 1 | | |
| > C₁₀-C₁₂ | 0 | 0.23 |
| > C₁₂-C₁₆ | 0.11 | 7.9 |
| > C₁₆-C₃₆ | 0. 63 | 180 |

| Amount of substituted benzene compounds in the soil sample of example 1 | | |
|---|---|---|
| > C₁₀-C₁₂ | 0.025 | - |
| > C₁₂-C₁₆ | 0.197 | - |
| > C₁₆-C₃₆ | 99.0 | - |

| Amount of terpene compounds in the soil sample of example 1 | | |
|---|---|---|
| > C₁₀-C₁₂ | 0 | - |
| > C₁₂-C₁₆ | 0.012 | - |
| > C₁₆-C₃₆ | 95.1 | - |

Table 1 shows that according to the GC-FID method the amount of C₁₀-C₁₂ aliphatics is small, but present, whereas according to the inventive method no such aliphatic compounds are present. For the higher aliphatics the difference is even more pronounced; the GC-FID method suggests relatively high amounts of these aliphatic compounds whereas the inventive methods suggests only small amounts. This example also reveals that substantial amounts of higher alkyl substituted benzenes as well as higher terpenes were present in the soil sample. These compounds could not be quantified separately according to the method of prior art.

### Example 2a

Again an authentic soil sample - this time having a different origin than the soil sample of example 1a - was subjected to the extraction treatment mentioned above and 1 µl was injected in the GC-MS instrument having the above settings.

When the sampling was accomplished a complete TIC (total ion chromatogram) was obtained. The TIC had the shape of a hill with peaks on top. Comparing the individual ions found in the MS with a selection of compounds in a database connected to the GC-MS instrument revealed very high abundances of the ion *m*/*z* = 57 compared to the abundances of the ions *m*/*z* = 93 and *m*/*z* = 91 respectively. Due to the fact that the peak abundances of the peaks in the "91-ion" and "93-ion" chromatogram were very low relative to the coincidal peaks of the "57-ion" chromatogram (as low as as 2.6 %) no deletion in step 5a) was performed (in this experiment the difference between the "57-ion" chromatogram and the TIC was not very significant due to the small abundances of the ions *m*/*z* = 93 and *m*/*z* = 91). Again the amounts of aliphatic hydrocarbon substances with different chain lengths in the soil sample extract were calculated as the sum of peak areas relative to the sum of peak areas of a standard solution of paraffin in a usual manner and finally the concentrations of aliphatic hydrocarbons in the soil sample were calculated. The results from example 2a is listed in table 2 below.

### Example 2b - Comparison

An identical sample preparation as in example 2a was used for analysis according to the GC-FID method with the conditions set forth above. The results from example 2b is listed in table 2 below.

**Table 2**

| Values for sample of example 2 | | |
|---|---|---|
| Chain length of aliphatic compounds | Example 2a Method according to the invention (mg/kg) | Example 2b GC-FID method (prior art) (mg/kg) |
| aliphatics > C₁₀-C₁₂ | 431 | 551 |
| aliphatics > C₁₂-C₁₆ | 2651 | 3201 |
| aliphatics > C₁₆-C₃₆ | 5673 | 7190 |

It appears from Table 2 that the results from the two methods of analysis are in good agreement, although small differences can be seen.

The above description of the invention reveals that it is obvious that it can be varied in many ways. Such variations are not to be considered a deviation from the scope of the invention, and all such modifications which are obvious to persons skilled in the art are also to be considered comprised by the scope of the succeeding claims.

## Claims

1. A method for quantitative assessment of anthropogenic compounds in a soil sample, wherein
1) an extract of the soil sample is subjected to a method for chromatographic separation of individual components in the extract; and
2) MS analyses are conducted on individual components found in step 1); and
3) the chromatograms corresponding to mass spectra in the MS analyses which show presence of
a) one or more of the ions *m*/*z* = 43, *m*/*z* = 57, *m*/*z* = 71, *m*/*z* = 85
b) one or more of the ions *m*/*z* = 77, *m*/*z* = 91, *m*/*z* = 105
c) one or more of the ions *m*/*z* = 68, *m*/*z* = 69, *m*/*z* = 93, *m*/*z* = 119
respectively are identified and peak integration of each of these chromatograms are performed; and
4) peak areas and retention times for individual peaks in each of the chromatograms
a) identified in step 3a)
b) identified in step 3b)
c) identified in step 3c)
respectively are listed numerically; and
5a) one or more peaks in the chromatogram identified in step 3a) which with respect to retention time coincide with one or more of the peaks corresponding to one or more of the ions set forth in step 3b) and/or step 3c) are deleted from the list obtained in step 4a);
5b) the concentration of aliphatic compounds in the soil sample extract is calculated as the sum of peak areas obtained from the revised list obtained in step 5a) relative to the sum of peak areas of a standard having a known concentration, and the concentration of aliphatic compounds in the soil sample is calculated on the basis of this; and/or
6) the concentration of alkyl substituted benzene compounds in the soil sample extract is calculated as the sum of peak areas obtained from the list obtained in step 4b) relative to the sum of peak areas of a standard having a known concentration, and the concentration of alkyl substituted benzene compounds in the soil sample is calculated on the basis of this; and/or
7a) one or more peaks in the chromatogram identified in step 3b) which with respect to retention time coincide with one or more of the peaks corresponding to one or more of the ions set forth in step 3c) are deleted from the list obtained in step 4b);
7b) the concentration of alkyl substituted benzene compounds not comprising terpene moieties present in the soil sample extract is calculated as the sum of peak areas obtained from the revised list obtained in step 7a) relative to the sum of peak areas of a standard having a known concentration, and the concentration of alkyl substituted benzene compounds not comprising terpene moieties present in the soil sample is calculated on the basis of this; and/or
8) the concentration of terpene compounds in the soil sample extract is calculated as the sum of peak areas obtained from the list obtained in step 4c) relative to the sum of peak areas of a standard and the concentration of terpene compounds in the soil sample is calculated on the basis of this.

2. A method according to claim 1 wherein the integration of the chromatogram identified in step 3a) is performed on a valley-to-valley basis within specific range/ranges of retention times for which range/ranges the curve representing the chromatogram identified in step 3a) and at least one of the curves representing the chromatogram identified in step 3b or 3c) appear to comprise "hills" with peaks on top provided that the curves representing these chromatograms appear to have essentially the same curve form within this/these range/ranges of retention times.

3. A method according to claim 1 or 2, wherein the soil sample is extracted with a solvent selected from the group comprising pentane, dichloromethane, toluene, hexane, ethyl acetate, acetone and mixtures of these.

4. A method according to any one of the preceding claims, wherein in step 3), only:
the chromatograms corresponding to mass spectra in the MS analyses which show presence of
a) one or two of the ions *m*/*z* = 57, *m*/*z* = 71
b) one or two of the ions *m*/*z* = 91, *m*/*z* = 105
c) one or two of the ions = 93, *m*/*z* = 119
are identified

5. A method according to claim 4, wherein in step 3), only:
the chromatograms corresponding to mass spectra in the MS analyses which show presence of
a) the ion *m*/*z* = 57
b) the ion *m*/*z* = 91
c) the ion *m*/*z* = 93
are identified

6. A method according to any one of the preceding claims, wherein the chromatographic separation is carried out by means of gas chromatography.

7. A method according to any of the preceding claims, wherein the ionisation in the MS instrument is performed by means of electron impact (EI).

8. A method according to any of the preceding claims wherein in step 5a) only those peaks corresponding to peaks in the chromatogram identified in step 3a) that have coincidal peaks in the chromatogram identified in step 3b) and/or 3c) having abundances of at least 10% the value of the corresponding peak in the chromatogram identified in step 3a) respectively are deleted from the list obtained in step 4).
